# EUROPÄISCHE PATENTSCHRIFT

(11) **EP 1 055 407 B1**
(45) Veröffentlichungstag und Bekanntmachung des Hinweises auf die Patenterteilung: **15.08.2012**
(21) Anmeldenummer: 00106667.9
(22) Anmeldetag: 29.03.2000
(51) Int. Cl.: A61Q 5/06, A61K 8/86

(54) **Haarstylingöl**
Hair styling oil
Huile pour la mise en forme des cheveux

(30) Priorität: 28.05.1999 DE 19924705
(43) Veröffentlichungstag der Anmeldung: 29.11.2000
(73) Patentinhaber: Wella GmbH, 65825 Schwalbach am Taunus (DE)
(72) Erfinder: Karlen, Thomas, Dr., 4056 Basel (CH); Kalbfleisch, Axel, 64295 Darmstadt (DE); Franzke, Michael, Dr., 64380 Rossdorf (DE); Borcard, Chantal, 1697 La Joux (CH)
(74) Vertreter: Herzog, Fiesser & Partner

(56) Entgegenhaltungen:
- EP-A- 0 445 659
- EP-A- 0 919 219
- US-A- 5 597 551

## Beschreibung

Gegenstand der Erfindung ist ein Haarbehandlungsmittel, welches die Konsistenz eines Haaröls aufweist, ohne hydrophobe Inhaltsstoffe zu enthalten und welches einen Fettsäureglyceridpolyalkylenglykolether oder einen Fettsäurepartialglyceridpolyalkylenglykolether mit mindestens 30 Alkylenglykoleinheiten, ein weiteres nichtionisches Tensid und einen Verdicker enthält.

Schon seit langem beschäftigt sich die Forschung auf dem Gebiet der Haarbehandlungsmittel mit Zubereitungen in Form von Ölen, welche zur erleichterten Formgebung und zur Erhöhung des Glanzes und des Haltes der Frisur dienen. Zur Erzielung der formgebenden und glanzerzeugenden Eigenschaften werden in den klassischen Haarölen normalerweise hydrophobe Öle im üblichen Sinn, d.h. natürliche, tierische bzw. pflanzliche Öle, Mineralöle, höhere Kohlenwasserstoffe (z.B. Paraffine), synthetische Öle oder Siliconöle verwendet. Das Problem bei dieser Art von Ölen liegt in der hohen Haarbelastung und in der schlechten Auswaschbarkeit dieser hydrophoben Substanzen. Bei wasserhaltigen Zubereitungen stellt sich zusätzlich das Problem der Stabilisierung der Formulierung, die zum Auftrennen in eine wäßrige und in eine lipophile Phase neigt.

Es besteht die Möglichkeit, nichtionische Tenside als Glanz- und Formbarkeitsgeber zu verwenden, wie zum Beispiel Fettalkoholethoxylate und Fettsäureethoxylate. Diese nichtionischen Tenside vermitteln jedoch üblicherweise nicht den Haargriff und den Haarglanz, welchen der Anwender von einem Haaröl erwartet. Die Verwendung von hohen Anteilen an nichtwässrigen Lösemitteln wie höheren Alkoholen, Glycerin oder Propylenglykol verbessern zwar den Glanz, verschlechtern aber die Formbarkeit der behandelten Haare.

Es stellte sich somit die Aufgabe, ein Haarbehandlungsmittel mit der Konsistenz und den positiven Anwendungseigenschaften eines Haaröls zur Verfügung zu stellen, welches aber die negativen, auf einen hohen Gehalt an hydrophoben Stoffen zurückzuführenden Eigenschaften nicht aufweist. Das Mittel soll insbesondere die Formbarkeit, die Kontrolle und den Glanz des Haares erhöhen, einfach handhabbar, gut in das Haar einarbeitbar und gut wieder auswaschbar sein.

Gelöst wird die Aufgabe durch die Verwendung eines speziellen, hochethoxylierten nichtionischen Tensids, welches bereits in geringen Mengen zu einem Mittel führt, das die Anmutung eines Öls aufweist, sich ausgezeichnet ins Haar einarbeiten läßt und der Frisur eine gute Formbarkeit und einen langanhaltenden Glanz verleiht sowie dem Haar einen langanhaltenden weichen Griff verleiht. Überraschenderweise wurde gefunden, daß das erfindungsgemäße Mittel die Anmutung eines Öls aufweist, obwohl seine Hauptbestandteile keine Öle darstellen und sich das Mittel einfach und rückstandslos wieder auswaschen läßt.

Gegenstand der Erfindung ist daher ein Haarbehandlungsmittel mit den rheologischen Eigenschaften eines Öls, bestehend aus nicht-hydrophoben Inhaltsstoffen und mit einem Gehalt an
(A) mindestens einem Fettsäureglyceridpolyalkylenglykolether oder einem Fettsäurepartialglyceridpolyalkylenglykolether mit mindestens 30 Alkylenglykoleinheiten,
(B) mindestens einem zusätzlichen, von (A) verschiedenen Tensid und
(C) mindestens einem Verdicker,
wobei das Mittel frei ist von Inhaltsstoffen in einer Menge, die ein Aufschäumen der Zusammensetzung vor oder während der Anwendung bewirken und eine Viskosität von 200 bis 4000 mm²/s bei 20°C aufweist. Bei den Partialglyceriden kann es sich um Mono- oder Diglyceride oder um Mischungen aus Mono- und Diglyceriden handeln. Nicht-hydrophobe Inhaltsstoffe im Sinne dieser Anmeldung sind insbesondere hydrophile und amphiphile Inhaltsstoffe. Das erfindungsgemäße Haarbehandlungsmittel enthält insbesondere anionische, kationische oder amphotere Tenside höchstens bis zu einem Gehalt, bei dem die Einarbeitung des Haarbehandlungsmittels ins Haar nicht zu einem Aufschäumen führt und ist frei von Treibmitteln oder mechanischen Vorrichtungen, welche ein Aufschäumen des Produktes bei der Entnahme bewirken.

Ein weiterer Gegenstand der Erfindung ist die Verwendung eines Fettsäureglyceridpolyalkylenglykolethers oder eines Fettsäurepartialglyceridpolyalkylenglykolethers mit 30 bis 1000 Alkylenglykoleinheiten zur Herstellung eines Haarbehandlungsmittels mit den rheologischen Eigenschaften eines Öls.

Unter den rheologischen Eigenschaften eines Öls im Sinne dieser Anmeldung wird eine Konsistenz verstanden, die zähflüssiger ist als Wasser aber im Gegensatz zu einem Gel fließfähig und formunbeständig ist, d.h. von einer schrägen Oberfläche bei einer Temperatur von ca. 25°C abläuft. Die Viskosität beträgt von 200 bis 4000 mm²/s bei 25 °C, besonders bevorzugt von 1000 bis kleiner 4000 bei 25 °C, gemessen mit einem Rotationsviskosimeter Haake VT-550 bei 25 °C und einem Schergefälle von 100 s⁻¹.

Das erfindungsgemäße Mittel enthält Komponente (A) vorzugsweise zu 0,1 bis 30 Gewichtsprozent, besonders bevorzugt zu 0,3 bis 20 beziehungsweise 0,5 bis 12 Gewichtsprozent. Komponente (A) ist vorzugsweise ausgewählt aus Verbindungen der allgemeinen Formel (I)

R¹-(OA)ₓ-O-CH₂-CH[O-(AO)_{y}-R²]-CH₂-O-(AO)_{z}-R³ (I)

wobei R¹, R² und R³ unabhängig voneinander ausgewählt sind aus H und gesättigten oder ungesättigten, verzweigten oder unverzweigten C₆- bis C₂₂-Acylgruppen, die gegebenenfalls mit einer oder mehreren Hydroxygruppen substituiert sein können, wobei mindestens einer der Substituenten R¹, R² und R³ eine C₆- bis C₂₂-Acylgruppe ist; A eine Alkylengruppe mit zwei oder drei Kohlenstoffatomen ist; x,y und z Zahlen zwischen 0 und 1000 sind, wobei die Summe x+y+z von 30 bis 1000, vorzugsweise 30 bis 500, besonders bevorzugt 70 bis 250 beträgt. Besonders bevorzugt sind Verbindungen, bei denen R¹ gleich H ist, R² ausgewählt ist aus H und C₆- bis C₂₂-Acylgruppen, R³ eine C₆- bis C₂₂-Acylgruppe bedeutet, A eine Ethylengruppe ist und x und y gleich 0 sind.

Beispiele für geeignete Verbindungen der Komponente (A) sind Polyethylenglykol(30)-glycerylcocoat, Polyethylenglykol(80)-glycerylcocoat, Polyethylenglykol(80)-glyceryltallowat, Polyethylenglykol(120)-glycerylstearat, Polyethylenglykol(200)-glycerylstearat, Polyethylenglykol(200)-glyceryltallowat, hydriertes Polyethylenglykol(200)-glycerylpalmitat. Besonders bevorzugt ist hydriertes Polyethylenglykol(200)-glycerylpalmitat.

Komponente (B) des erfindungsgemäßen Mittels ist mindestens ein weiteres, vorzugsweise nicht-ionisches Tensid in einer Menge von vorzugsweise 0,1 bis 30 Gewichtsprozent, besonders bevorzugt von 0,5 bis 20 Gewichtsprozent. Art und Menge des Tensids werden so gewählt, daß die erfindungsgemäße Zusammensetzung weder bei der Entnahme aus der Verpackung noch bei der Aufbringung und Einarbeitung ins Haar aufschäumt, sondern sich bei der Einarbeitung im wesentlichen so verhält wie ein Haaröl im klassischen Sinne. Es können nichtionische, anionische, kationische oder amphotere Tenside verwendet werden, wobei jedoch nicht-ionische Tenside bevorzugt werden. Geeignete nicht-ionische Tenside sind z.B. ethoxylierte Fettsäuren mit 10 bis 26 Kohlenstoffatomen, ethoxylierte Fettalkohole mit 10 bis 26 Kohlenstoffatomen, alkoxylierte Fettsäureester ethoxyliertes hydriertes oder nicht hydriertes Rizinusöl, Glyceridalkoxylate, Fettsäureglyceridpolyalkylenglykolether oder Fettsäurepartialglyceridpolyalkylenglykolether mit jeweils weniger als 30 Alkylenglykoleinheiten wie beispielsweise Polyethylenglykol(7)-glycerylcocoat, Polyglykolamide, ethoxylierte oder nicht-ethoxylierte Fettsäurepolyolester und Alkylpolyglycoside. Der Ethoxylierungsgrad von ethoxylierten Tensiden ist dabei vorzugsweise größer als 3.

Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können oder oxethyliertes Lanolin. Geeignete ethoxylierte Fettsäuren sind beispielsweise Polyethylenglykol(75)-laurat, Polyethylenglykol(90)-stearat, Polyethylenglykol(120)-stearat, Polyethylenglykol(120)-propylenglykolstearat, Polyethylenglykol(150)-dilaurat oder Polyethylenglykol(175)-distearat.

Geeignete Polyolester sind z.B. ethoxylierte und nichtethoxylierte Zuckerester, Sorbitolester und Glycerinester. Beispiele sind ethoxylierte Sorbitanfettsäureester mit der INCI-Bezeichnung Polysorbate, nicht ethoxylierte Sorbitanester wie Sorbitanlaurat, Sorbitanpalmitat etc., nicht-ethoxylierte Glycerinester wie Glycerylstearat sowie Glucosederivate wie Polyethylenglykol(120)-methylglucosedioleat und Alkylpolyglucoside wie z.B. Coco-Glucoside, Lauryl-Glucoside oder Decyl-Glucoside.

Die verdickende Komponente (C) ist in einer solchen Menge enthalten, daß sich für die Gesamtzusammensetzung die rheologischen Eigenschaften eines Öls ergeben und sich insbesondere eine Viskosität von vorzugsweise 200 bis 4000 mm²/s bei 25 °C ergibt, besonders bevorzugt von 1000 bis kleiner 4000 mm²/s bei 25 °C, gemessen mit einem Rotationsviskosimeter Haake VT-550 bei 25 °C und einem Schergefälle von 100 s⁻¹. Die hierfür benötigte genaue Einsatzmenge hängt von der Art des gewählten Verdickers ab. Typische Einsatzkonzentrationen sind von 0,05 bis 3 Gewichtsprozent, besonders bevorzugt von 0,1 bis 1 Gew.%.

Als verdickende Komponente (C) werden natürliche oder synthetische Polymere mit verdickender Wirkung oder anorganische Verdicker eingesetzt. Besonders bevorzugt sind nichtionische und anionische Polymere. Beispiele für Verdicker, die in dem erfindungsgemäßen Mittel enthalten sein können, sind Homopolymere der Acrylsäure, die unter der INCI-Bezeichnung Carbomer bekannt sind, Acrylsäure/Acrylamid Copolymere, Sclerotium Gum oder Copolymere der Acrylsäure oder der Methacrylsäure. Beispiele für geeignete Copolymere sind beispielsweise Acryl- oder Methacrylsäure/Acryl- oder Methacrylsäurepolyethoxyalkylester Copolymere (z.B. Acrylates/ Steareth-20 Methacrylate Copolymer), Acryl- oder Methacrylsäure/Polyethoxyalkylallylether Copolymere (z.B. Steareth-10 Allyl Ether/Acrylates Copolymer) oder Acryl- oder Methacrylsäure/Itaconsäurepolyethoxyalkylester Copolymere (z.B. Acrylates/Steareth-20 Itaconate Copolymer und Acrylates/Ceteth-20 Itaconate Copolymer).

Desweiteren können natürliche Verdicker eingesetzt werden wie Cellulosen oder Cellulosederivate, Tragacanth, Guar Gum, Karaya Gum, Carrageenan, Xanthan, Stärken oder Stärkederivate. Cellulosederivate sind z.B. ethoxylierte Cellulosen, Hydroxyethylcellulose, Hydroxymehtylcellulose, Methylcellulose. Guarderivate sind beispielsweise Guar Hydroxypropyltrimonium Chloride oder Hydroxypropylguar. Anorganische Verdicker sind beispielsweise Bentonite oder Hektorite. Besonders bevorzugte Verdicker sind Hydroxyethylcellulose und vernetzte Polyacrylsäuren oder deren Gemische.

Bevorzugte Ausführungen des erfindungsgemäßen Mittels sind im wesentlichen frei von kationischen Verdickern, da diese sich durch ihre Substantivität zum Haar nur erschwert vollständig entfernen lassen.

Bevorzugte Ausführungen des erfindungsgemäßen Mittels sind im wesentlichen frei von Verdickern mit emulgierenden Eigenschaften, da diese in Verbindung mit erfindungsgemäßen nichtionischen Tensiden zu einer synergistischen Steigerung der Viskosität führen können, womit die Anmutung des Mittels nicht mehr derjenigen eines Öls entspricht. Emulgierende verdickende Polymere sind quervernetzte Polymere, welche langkettige, gegebenenfalls ethoxylierte, Kohlenwasserstoffseitenketten beinhalten.

Das erfindungsgemäße Mittel liegt vorzugsweise in einer wässrigen oder in einer wässrig-alkoholischen Lösung vor. Die Viskosität der Lösung ist vorzugsweise kleiner als 4000 mm²/s bei 20 °C. Als Alkohole können insbesondere die für kosmetische Zwecke üblicherweise verwendeten niederen einwertigen Alkohole mit 1 bis 4 Kohlenstoffatomen wie zum Beispiel Ethanol oder Isopropanol aber auch die niederen mehrwertigen Alkohole wie Propylenglykole oder Glycerin enthalten sein. Die Lösungsmittel liegen in einer Menge von 0,01 bis 50 Gewichtsprozent, bevorzugt in einer Menge von 2 bis 30 Gewichtsprozent vor.

Wasserunlösliche Lösungsmittel wie beispielsweise unverzweigte oder verzweigte Kohlenwasserstoffe wie Pentan, Hexan, Isopentan und zyklische Kohlenwasserstoffe wie Cyclopentan und Cyclohexan sowie Paraffine oder Isododecan und hydrophobe synthetische oder natürliche Öle sind vorzugsweise nicht enthalten oder nur in solchen Mengen, die die Auswaschbarkeit nicht negativ beeinflussen.

In einer bevorzugten Ausführungsform enthält das erfindungsgemäße Haarbehandlungsmittel als weiteren zusätzlichen Bestandteil (D) mindestens ein filmbildendes, haarfestigendes Polymer in einer Menge von vorzugsweise 0,01 bis 10 Gewichtsprozent, besonders bevorzugt von 0,1 bis 8 Gewichtsprozent. Das Polymer kann synthetischen oder natürlichen Ursprungs und nichtionischer, anionischer, kationischer oder amphoterer Natur sein. Die haarfestigenden Polymere können einzeln oder im Gemisch eingesetzt werden.

Unter filmbildenden, haarfestigenden Polymeren werden erfindungsgemäß solche Polymere verstanden, die bei Anwendung in 0,1 bis 5%iger wäßriger, alkoholischer oder wäßrig-alkoholischer Lösung in der Lage sind, auf dem Haar einen Polymerfilm abzuscheiden und auf diese Weise das Haar zu festigen.

Geeignete synthetische, nichtionische, filmbildende, haarfestigende Polymere sind zum Beispiel Homo- oder Copolymere, welche aus mindestens einem nichtionischen Monomer aufgebaut sind. Nichtionische Monomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1-bis C3-Alkylgruppen sind. Geeignete synthetische, nichtionische, filmbildende, haarfestigende Polymere sind zum Beispiel Homopolymere des Vinylpyrrolidons sowie Homopolymere des N-Vinylformamids. Weitere geeignete synthetische filmbildende, nichtionische haarfestigende Polymere sind zum Beispiel Copolymerisate aus Vinylpyrrolidon und Vinylacetat, Terpolymere aus Vinylpyrrolidon, Vinylacetat und Vinylpropionat, Polyacrylamide, Polyvinylalkohole oder Polyethylenglykole mit einem Molekulargewicht von 800 bis 20.000 g/mol. Geeignete anionische Polymere sind synthetische Homooder Copolymere mit neutralisierbare Säuregruppen enthaltenden Monomereinheiten, welche gegebenenfalls mit Comonomeren, die keine Säuregruppen enthalten, copolymerisiert sind. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Aldehydocarbonsäuren oder Ketocarbonsäuren. Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1-bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes anionisches, natürliches Polymer ist beispielsweise teilweise oder vollständig neutralisierter Schellack.

Bevorzugte Polymere mit Säuregruppen sind vernetzte oder unvernetzte Crotonsäure/Vinylacetat Copolymere, Terpolymere aus Vinylacetat, Crotonsäure und Polyethylenoxid, Vinylpyrrolidon/Vinylacrylat Copolymere, Terpolymere aus Acrylsäure, Alkylacrylat und N-Alkylacrylamid, insbesondere Acrylsäure/ Ethyläcrylat/N-t-Butylacrylamid Terpolymere, Terpolymere aus Vinylacetat, Crotonat und Vinylalkanoat, insbesondere Vinylacetat/Crotonat/Vinylneodecanoat Copolymere sowie Methylvinylether/Maleinsäureanhydrid Copolymere und deren Monoester.

Eine weitere Klasse von geeigneten anionischen Polymeren sind anionische Polyurethane. Bevorzugte Polyurethane sind dadurch gekennzeichnet, daß sie (a) endständige Säuregruppen besitzen, die beispielweise über Aminosulfonsäuren oder Aminocarbonsäuren eingeführt wurden, (b) gegebenenfalls weitere freie Carbonsäuregruppen enthalten, die durch Einpolymerisieren von Carbonsäurediolen wie beispielsweise Dimethylolpropansäure als Comonomere eingeführt wurden und (c) Polyurethansequenzen enthalten, die aus Polyesterdiolen und Diisocyanaten wie beispielsweise Alkylendiisocyanaten oder Isophorondiisocyanat gebildet wurden. Geeignet ist beispielsweise Luviset^{®} PUR der Firma BASF/Deutschland.

Die anionischen Polymere liegen im erfindungsgemässen Mittel teilweise oder vollständig mit einem kosmetisch verträglichen Neutralisationsmittel neutralisiert vor. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH u.a..

Geeignete natürliche filmbildende Polymere oder daraus durch chemische Umwandlung hergestellte Derivate sind zum Beispiel Chitosan, Hydroxyalkylchitosane, Hydroxyalkylchitine, Polysaccharide oder Gemische aus Oligo-, Mono- und Disacchariden, chinesisches Balsamharz (Kolophonium), Cellulosederivate wie Hydroxypropylcellulose mit einem Molekulargewicht von 30.000 bis 50.000 g/mol, oder Schellack in neutralisierter oder unneutralisierter Form.

Geeignete filmbildende kationische Polymere sind dadurch gekennzeichnet, daß sie aus mindestens einer Monomerart aufgebaut sind, die kationische oder kationisierbare Gruppen, vorzugsweise primäre, sekundäre, tertiäre oder quaternäre Stickstoffgruppen enthält. Geeignete ammoniumsubstituierte Vinylmonomere sind zum Beispiel Trialkylmethacryloxyalkylammonium, Trialkylacryloxyalkylammonium, Dialkyldiallylammonium, quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, welches am heterocyclischen Ring mit bis zu 3 C1- bis C12-Alkylresten substituiert sein kann, Alkylvinylpyridinium, oder Alkylvinylpyrrolidon Salze. Geeignete aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, N-Vinylimidazol, welches am Ring mit bis zu 3 C1- bis C12-Alkylresten substituiert sein kann. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Die kationischen bzw. basischen Monomere können mit nicht-kationischen bzw. nicht-basischen Comonomeren copolymerisiert sein.

Geeignete kationische Polymere sind zum Beispiel Polyvinylpyrrolidon/Dimethylaminoethylmethacrylat Copolymere, Terpolymere aus Vinylpyrrolidon, Dimethylaminoethylmethacrylat und Vinylcaprolactam, Vinylpyrrolidon/Methacrylamidopropyltrimethylammoniumchlorid Copolymere, Copolymere des Vinylpyrrolidons mit quaternierten Derivaten des Dialkylaminoalkylacrylats oder -methacrylats, z.B. das mit Diethylsulfat quaternierte Vinylpyrrolidon/Dimethylaminoethylmethacrylat Copolymer, Copolymerisate des Vinylpyrrolidons mit Vinylimidazoliummethochlorids, Polymere von Dimethyldiallylammoniumsalzen und deren Copolymere mit Estern oder Amiden von Acryl- oder Methacrylsäure, Kondensationsharze aus Polyglykolen und Polyaminen, kationisch derivatisierte Silikone, z.B. diquaternäre Polydimethylsiloxane, kationisch derivatisierte Proteinhydrolysate und quaternisierte Cellulose- oder Guarderivate.

Kationische Polymere, welche basische Gruppen enthalten, können mit organischen oder anorganischen Säuren ganz oder teilweise neutralisiert werden.

Geeignete amphotere Polymere sind Polymere, welche sowohl kationische oder durch Protonierung kationisierbare Gruppen als auch anionische oder durch Deprotonierung anionisierbare Gruppen enthalten. Kationische Gruppen sind beispielsweise quaternäre Amingruppen, kationisierbare Gruppen sind beispielsweise primäre, sekundäre oder tertiäre Amingruppen. Anionische Gruppen sind beispielsweise Carboxylat-, Sulfat-, Sulfonat-, Phosphat - oder Phosphonatgruppen. Anionisierbare Gruppen sind beispielsweise die protonierten Formen der genannten anionischen Gruppen.

Geeignete amphotere Polymere sind z.B. Copolymere aus Octylacrylamid, t-Butylaminoethylmethacrylat und zwei oder mehr Monomeren, bestehend aus Acrylsäure, Methacrylsäure oder deren Estern, wobei mindestens eine Monomerart eine Säuregruppe enthält. Weitere Beispiele sind Copolymere von Acrylsäure, Methylacrylat und Methacrylamidopropyltrimethylammoniumchlorid (INCI: Polyquaternium-47), Copolymere aus Acrylamidopropyltrimoniumchlorid und Acrylaten oder Copolymere aus Acrylamid, Acrylamidopropyltrimoniumchlorid, 2-Amidopropylacrylamidsulfonat und DMAPA (INCI: Polyquaternium-43).

Selbstverständlich kann das erfindungsgemäße Mittel auch weitere übliche kosmetische Zusätze, wie Parfümöle in einer Menge von vorzugsweise 0,01 bis 5 Gewichtsprozent; Trübungsmittel wie z.B. Ethylenglykoldistearat, Styrol/PVP Copolymere oder Polystyrole in einer Menge von vorzugsweise 0,01 bis 5 Gewichtsprozent; Feuchthaltemittel, Farbstoffe, Lichtschutzmittel, Antioxidantien, Glanzgeber und Konservierungsstoffe in einer Menge von vorzugsweise 0,01 bis 10 Gewichtsprozent enthalten.

Das erfindungsgemäße Mittel bewirkt eine Verbesserung der Formbarkeit, einen langanhaltenden Glanz und einen weichen Griff des Haars und besitzt die Anmutung eines Öls ohne daß seine Hauptbestandteile aus Ölen im klassichen Sinne bestehen.

Außerdem ist mit dem erfindungsgemäßen Mittel die Erzeugung eines sehr schönen Nasseffektes, auch Wet-Look genannt, möglich. Der Wet Look-Effekt zeichnet sich dadurch aus, daß er über längere Zeit bestehen bleibt. Eine hierfür besonders geeigenete, bevorzugte Ausführungsform enthält
(A) mindestens einen Polyethylenglykolglycerylfettsäureester mit mindestens 30 Ethylenglykoleinheiten,
(B) ethoxyliertes, hydriertes Rizinusöl und
(C) mindestens einen Verdicker ausgewählt aus Hydroxyethylcellulose, vernetzter Polyacrylsäure oder deren Gemisch.

Die nachfolgenden Beispiele sollen den Gegenstand der Erfindung näher erläutern:

### Beispiele

### Beispiel 1: Haarbehandlungsmittel zur erleichterten Formgebung

| | |
|---|---|
| 0,75 g | hydriertes Polyethylenglykol(200) - glycerylpalmitat |
| 0,25 g | Polyethylenglykol(7)-glycerylcocoat |
| 9,0 g | hydriertes Rizinusöl, ethoxyliert mit 40 mol Ethylenoxid |
| 0,50 g | Hydroxyethylcellulose |
| 0,01 g | Aminomethylpropanol |
| ad 100 g | Wasser |

### Beispiel 2: Haarbehandlungsmittel zur erleichterten Formgebung

| | |
|---|---|
| 5,25 g | hydriertes Polyethylenglykol(200) - glyceryltallowat |
| 1,75 g | Polyethylenglykol(7)-glycerylcocoat |
| 1,50 g | Laureth-4 |
| 0,50 g | Carbomer K mit Aminomethylpropanol neutralisiert |
| ad 100 g | Wasser |

### Beispiel 3: Haarbehandlungsmittel mit Pflegeeffekt

| | |
|---|---|
| 0,7 g | hydriertes Polyethylenglykol(200) - glycerylpalmitat |
| 9,0 g | hydriertes Rizinusöl, ethoxyliert mit 35 mol Ethylenoxid |
| 3,0 g | Cetyltrimethylammoniumchlorid |
| 0,5 g | Cellulose Gum |
| ad 100 g | Wasser |

### Beispiel 4: Haarbehandlungsmittel mit Pflegeeffekt

| | |
|---|---|
| 5,25 g | hydriertes Polyethylenglykol(200) - glycerylpalmitat |
| 1,75 g | Polyethylenglykol(7)-glycerylcocoat |
| 0,9 g | hydriertes Rizinusöl, ethoxyliert mit 35 mol Ethylenoxid |
| 0,3 g | Glucosedecylether |
| 1,0 g | Amodimethicone |
| 0,5 g | Guar Gum |
| ad 100 g | Wasser |

### Beispiel 5: Haarbehandlungsmittel mit verstärktem Glanz und Naßeffekt

| | |
|---|---|
| 7,0 g | Polyethylenglykol(30)-glycerylcocoat |
| 10,0 g | Propylenglykol |
| 3,0 g | Laureth-4 |
| 0,6 g | Hydroxyethylcellulose |
| ad 100 g | Wasser |

### Beispiel 6: Haarbehandlungsmittel mit schnelltrocknendem Effekt

| | |
|---|---|
| 7,0 g | Polyethylenglykol(80)-glycerylcocoat |
| 3,0 g | Oleth-10 |
| 20,0 g | Ethanol |
| 0,7 g | Carbopol ETD 2020 mit Aminomethylpropanol neutralisiert |
| ad 100 g | Wasser |

### Beispiel 7: Haarbehandlungsmittel mit schnelltrocknendem Effekt

| | |
|---|---|
| 7,0 g | Polyethylenglykol(80)-glyceryltallowat |
| 9,0 g | hydriertes Rizinusöl, ethoxyliert mit 40 mol Ethylenoxid |
| 20,0 g | Ethanol |
| 0,3 g | Xanthan Gum |
| ad 100 g | Wasser |

### Beispiel 8: Haarbehandlungsmittel mit Festigung

| | |
|---|---|
| 7,0 g | hydriertes Polyethylenglykol(200) - glycerylpalmitat |
| 0,2 g | Polyoxyethylen(120)-Methylglucosedioleat |
| 3,0 g | Polysorbate 40 |
| 4,0 g | Polyvinylpyrrolidon (Luvsikol^{®} K 60, BASF) |
| 0,5 g | Carbopol 980 neutralisiert mit AMP |
| ad 100 g | Wasser |

### Beispiel 9: Haarbehandlungsmittel mit Festigung

| | |
|---|---|
| 5,25 g | hydriertes Polyethylenglykol(200) - glycerylpalmitat |
| 1,75 g | Polyethylenglykol(7)-glycerylcocoat |
| 3,0 g | Polysorbate 20 |
| 2,0 g | Vinylacetat/Crotonat Copolymer (Luviset^{®} CA-66, BASF) neutralisiert mit AMP |
| 0,5 g | Carbopol 980 neutralisiert mit AMP |
| ad 100 g | Wasser |

### Beispiel 10: Haarbehandlungsmittel mit Festigung

| | |
|---|---|
| 7,0 g | Polyethylenglykol(80)-glyceryltallowat |
| 3,0 g | Polyethylenglykol(400)monooleat |
| 4,0 g | Vinylpyrrolidon/Dimethylaminoethylmethacrylat Methosulfat Copolymer, 20%ig in Wasser (Gafquat^{®} 755 N, ISP) |
| 0,5 g | Methyl-hydroxyethyl-cellulose (Tylose^{®}) |
| ad 100 g | Wasser |

### Beispiel 11: Haarbehandlungsmittel mit Festigung

| | |
|---|---|
| 5,25 g | hydriertes Polyethylenglykol(200) - glyceryltallowat |
| 1,75 g | Polyethylenglykol(7)-glycerylcocoat |
| 9,0 g | hydriertes Rizinusöl, ethoxyliert mit 40 mol Ethylenoxid |
| 1,0 g | Octylacrylamid/Acrylat/Butylaminoethylmethacrylat Copolymer (Amphomer^{®}, National Starch) neutralisiert mit AMP |
| 0,2 g | Carbopol 980 neutralisiert mit AMP |
| 0,3 g | Hydroxyethylcellulose (Natrosol^{®} HHR) |
| ad 100 g | Wasser |

### Beispiel 12: Haarbehandlungsmittel mit Festigung

| | |
|---|---|
| 5,25 g | hydriertes Polyethylenglykol(200) - glyceryltallowat |
| 1,75 g | Polyethylenglykol(7)-glycerylcocoat |
| 3,0 g | Laureth-23 |
| 0,6 g | Chitosan |
| 0,5 g | Xanthan Gum |
| ad 100 g | Wasser |

## Patentansprüche

1. Haarbehandlungsmittel mit den rheologischen Eigenschaften eines Öls, bestehend aus nicht-hydrophoben Inhaltsstoffen und mit einem Gehalt an
(A) mindestens einem Fettsäureglyceridpolyalkylenglykolether oder einem Fettsäurepartialglyceridpolyalkylenglykolether mit mindestens 30 Alkylenglykoleinheiten,
(B) mindestens einem zusätzlichen, von (A) verschiedenen Tensid und
(C) mindestens einem Verdicker,
wobei das Mittel frei ist von Inhaltsstoffen in einer Menge, die ein Aufschäumen der Zusammensetzung vor oder während der Anwendung bewirken, und eine Viskosität von 200 bis 4000 mm²/s bei 20°C aufweist.

2. Mittel nach Anspruch 1, **dadurch gekennzeichnet, daß** Komponente (A) ausgewählt ist aus Verbindungen der allgemeinen Formel (I)
R¹-(OA)_{X}-O-CH₂-CH[O-(AO)_{Y}-R²]-CH₂-O-(AO)_{z}-R³ (I)
wobei R¹, R² und R³ unabhängig voneinander ausgewählt sind aus H und gesättigten oder ungesättigten, verzweigten oder unverzweigten C₆- bis C₂₂-Acylgruppen, wobei mindestens einer der Substituenten R¹, R² und R³ eine C₆- bis C₂₂-Acylgruppe ist; A eine Alkylengruppe mit zwei oder drei Kohlenstoffatomen ist; x,y und z Zahlen zwischen 0 und 1000 sind, wobei die Summe x+y+z von 30 bis 1000 beträgt.

3. Mittel nach einem der vorstehenden Ansprüche,
**dadurch gekennzeichnet, daß** R¹ gleich H ist, R² ausgewählt ist aus H und C₆- bis C₂₂-Acylgruppen, R³ eine C₆ - bis C₂₂-Acylgruppe bedeutet, A eine Ethylengruppe ist und x und y gleich 0 sind.

4. Mittel nach einem der vorstehenden Ansprüche,
**dadurch gekennzeichnet, daß** Komponente (A) hydriertes Glycerylpalmitat mit rund 200 Polyethylenglykoleinheiten ist.

5. Mittel nach einem der vorstehenden Ansprüche,
**dadurch gekennzeichnet, daß** es 0,1 bis 30 Gewichtsprozent der Komponente (A) enthält.

6. Mittel nach einem der vorstehenden Ansprüche,
**dadurch gekennzeichnet, daß** Komponente (B) ausgewählt ist aus nichtionischen Tensiden.

7. Mittel nach Anspruch 6, **dadurch gekennzeichnet, daß** Komponente (B) ausgewählt ist aus ethoxylierten Fettsäuren, ethoxylierten Fettalkoholen, Glyceridalkoxylaten, Fettsäureglyceridpolyalkylenglykolethern oder Fettsäurepartialglyceridpolyalkylenglykolethern mit jeweils weniger als 30 Alkylenglykoleinheiten, Polyglykolamiden, ethoxylierten und nicht-ethoxylierten Fettsäurepolyolestern und Alkylpolyglycosiden.

8. Mittel nach einem der vorstehenden Ansprüche, **dadurch gekennzeichnet, daß** es 0,1 bis 30 Gewichtsprozent der Komponente (B) enthält.

9. Mittel nach einem der vorstehenden Ansprüche,
**dadurch gekennzeichnet, daß** Komponente (C) ausgewählt ist aus nichtionischen und anionischen Polymeren oder deren Mischungen.

10. Mittel nach einem der vorstehenden Ansprüche,
**dadurch gekennzeichnet, daß** Komponente (C) in einer Menge von 0,05 bis 3 Gewichtsprozent enthalten ist.

11. Mittel nach einem der vorstehenden Ansprüche,
**dadurch gekennzeichnet, daß** das Lösungsmittel ausgewählt ist aus Wasser und ein- oder mehrwertigen Alkoholen mit 1 bis 4 Kohlenstoffatomen oder deren Mischung.

12. Mittel nach einem der vorstehenden Ansprüche,
**dadurch gekennzeichnet, daß** es als Komponente (D) zusätzlich ein filmbildendes, haarfestigendes Polymer enthält.

13. Mittel nach Anspruch 12, **dadurch gekennzeichnet, daß** es 0,01 bis 10 Gewichtsprozent der Komponente (D) enthält.

14. Verwendung eines Fettsäureglyceridpolyalkylenglykolethers oder eines Fettsäurepartialglyceridpolyalkylenglykolethers mit 30 bis 1000 Alkylenglykoleinheiten zur Herstellung eines Haarbehandlungsmittels mit den rheologischen Eigenschaften eines Öls.

## Claims

1. A hair treatment agent with the rheological properties of an oil, consisting essentially of non-hydrophobic ingredients and with a content of
(A) at least one fatty acid glyceride polyalkylene glycol ether or a fatty acid partial glyceride polyalkylene glycol ether with at least 30 alkylene glycol units,
(B) at least one additional surfactant different from (A) and
(C) at least one thickener,
wherein the agent is free of ingredients in a quantity that produces a foaming of the composition before or during the application and has a viscosity of 200 to 4000 mm²/s at 20°C.

2. The agent according to Claim 1, **characterized in that** component (A) is selected from compounds of the general formula (I)
R¹-(OA)_{X}-O-CH₂-CH[O-(AO)_{Y}-R²]-CH₂-O-(AO)_{Z}-R³ (I)
wherein R¹, R², and R³ are selected independent of one another from H and saturated or unsaturated, branched or unbranched C₆- to C₂₂-acyl groups, wherein at least one of the substituents R¹, R², and R³ is a C₆- to C₂₂-acyl group; A is an alkylene group with two or three carbon atoms; x, y, and z are numbers between 0 and 1000, wherein the sum x+y+z amounts to 30 to 1000.

3. The agent according to any one of the preceding claims, **characterized in that** R¹ is equal to H, R² is selected from H and C₆- to C₂₂-acyl groups, R³ means a C₆- to C₂₂-acyl group, A is an ethylene group, and x and y are equal to 0.

4. The agent according to any one of the preceding claims, **characterized in that** component (A) is hydrogenated glyceryl palmitate with around 200 polyethylene glycol units.

5. The agent according to any one of the preceding claims, **characterized in that** it contains 0.1 to 30 % by weight of component (A).

6. The agent according to any one of the preceding claims, **characterized in that** component (B) is selected from nonionic surfactants.

7. The agent according to claim 6, **characterized in that** component (B) is selected from ethoxylated fatty acids, ethoxylated fatty alcohols, glyceride alkoxylates, fatty acid glyceride polyalkylene glycol ethers, or fatty acid partial glyceride polyalkylene glycol ethers with respectively fewer than 30 alkylene glycol units, polyglycol amides, ethoxylated and non-ethoxylated fatty acid polyolesters, and alkyl polyglycosides.

8. The agent according to any one of the preceding claims, **characterized in that** it contains 0.1 to 30 % by weight of component (B).

9. The agent according to any one of the preceding claims, **characterized in that** component (C) is selected from nonionic and anionic polymers, or mixtures thereof.

10. The agent according to any one of the preceding claims, **characterized in that** component (C) is contained in a quantity from 0.05 to 3 % by weight.

11. The agent according to any one of the preceding claims, **characterized in that** the solvent is selected from water and monovalent or multivalent alcohols with 1 to 4 carbon atoms, or a mixture thereof.

12. The agent according to any one of the preceding claims, **characterized in that** it additionally contains a film-forming, hair-setting polymer as component (D).

13. The agent according to claim 12, **characterized in that** it contains 0.01 to 10 % by weight of component (D).

14. Use of a fatty acid glyceride polyalkylene glycol ether or a fatty acid partial glyceride polyalkylene glycol ether with 30 to 1000 alkylene glycol units to produce a hair treatment agent with the rheological properties of an oil.

## Revendications

1. Produit de traitement pour les cheveux, ayant les propriétés rhéologiques d'une huile, constitué de composants non hydrophobes et contenant
(A) au moins un éther de glycéride polyalkylène glycol d'acide gras ou un éther partiel de glycéride polyalkylène glycol d'acide gras avec au moins 30 unités d'alkylène glycol,
(B) au moins un agent tensioactif supplémentaire différent de (A) et
(C) au moins un agent épaississant,
dans lequel le produit ne contient pas de composants en une quantité, qui entraîne que la composition mousse avant ou pendant l'utilisation et présente une viscosité comprise entre 200 et 4000 mm²/s à 20°C.

2. Produit selon la revendication 1, **caractérisé en ce que** le composant (A) est sélectionné en rapport avec la formule générale (I)
R¹-(OA)_{X}-O-CH₂-CH[O-(AO)_{Y}-R²]-CH_{Z}-O-(AO)_{Z}-R³ (I)
dans laquelle R¹, R² et R³ sont sélectionnés de manière indépendante les uns des autres parmi des groupes acyles en C₆ à C₂₂ ramifiés ou non ramifiés, saturés ou insaturés et H, dans lequel au moins un des produits de substitution R¹, R² et R³ est un groupe acyle en C₆ à C₂₂ ; A est un groupe alkylène avec deux ou trois atomes de carbone ; les chiffres x, y et z sont compris entre 0 et 1000, dans lequel la somme x+y+z est comprise entre 30 et 1000.

3. Produit selon l'une des revendications précédentes, **caractérisé en ce que** R¹ est égal à H, R² est sélectionné parmi H et des groupes acyles en C₆ à C₂₂, R³ est un groupe acyle en C₆ à C₂₂, A est un groupe éthylène et x et y sont égaux à 0.

4. Produit selon l'une des revendications précédentes, **caractérisé en ce que** le composant (A) est un palmitate de glycérol hydrogéné avec environ 200 unités de polyéthylène glycol.

5. Produit selon l'une des revendications précédentes, **caractérisé en ce qu'**il comprend entre 0,1 et 30 pourcent en poids du composant (A).

6. Produit selon l'une des revendications précédentes, **caractérisé en ce que** le composant (B) est sélectionné parmi des agents tensioactifs non ioniques.

7. Produit selon la revendication 6, **caractérisé en ce que** le composant (B) est sélectionné parmi des acides gras éthoxylés, des alcools gras éthoxylés, des alkoxylates de glycérol, des éthers de glycéride polyalkylène glycol d'acides gras ou des éthers partiels de glycéride polyalkylène glycol d'acides gras, avec respectivement moins de 30 unités d'alkylène glycol, amides de polyglycol, des esters de polyol d'acides gras éthoxylés et non éthoxylés et polyglycoside d' aklyle.

8. Produit selon l'une des revendications précédentes, **caractérisé en ce qu'**il comprend entre 0,1 et 30 pourcent en poids du composant (B).

9. Produit selon l'une des revendications précédentes, **caractérisé en ce que** le composant (C) est sélectionné parmi des polymères non ioniques et anioniques ou leurs mélanges.

10. Produit selon l'une des revendications précédentes, **caractérisé en ce que** le composant (C) est présent en une quantité comprise entre 0,05 et 3 pourcent en poids.

11. Produit selon l'une des revendications précédentes, **caractérisé en ce que** le solvant est sélectionné parmi de l'eau et un alcool monovalent ou polyvalent avec 1 à 4 atomes de carbone ou leurs mélanges.

12. Produit selon l'une des revendications précédentes, **caractérisé en ce qu'**il comprend, en outre, en tant que composant (D), un polymère de formation de film pour la fixation des cheveux.

13. Produit selon la revendication 12, **caractérisé en ce qu'**il comprend entre 0,01 et 10 pourcent en poids du composant (D).

14. Utilisation d'un éther de glycéride polyalkylène glycol d'acide gras ou un éther partiel de glycéride polyalkylène glycol d'acide gras avec entre 30 et 1000 unités d'alkylène glycol pour la fabrication d'un produit de traitement pour les cheveux ayant les propriétés rhéologiques d'une huile.
